(19) Europäisches Patentamt
European Patent Office
Office européen des brevets

(11) Numéro de publication : **0 054 847**
**B1**

(12) # FASCICULE DE BREVET EUROPÉEN

(45) Date de publication du fascicule du brevet : **24.04.85**

(21) Numéro de dépôt : **81110303.5**

(22) Date de dépôt : **10.12.81**

(51) Int. Cl.[4] : **C 07 C149/26**, C 11 B 9/00, A 23 L 1/226, A 23 L 1/235, A 23 L 2/02, A 24 B 15/34, A 61 K 7/46

(54) **Utilisation de composés terpéniques soufrés en tant qu'ingrédients parfumants et aromatisants.**

(30) Priorité : **23.12.80 CH 9513/80**

(43) Date de publication de la demande :
**30.06.82 Bulletin 82/26**

(45) Mention de la délivrance du brevet :
**24.04.85 Bulletin 85/17**

(84) Etats contractants désignés :
**CH DE FR GB IT LI NL SE**

(56) Documents cités :
**DE-A- 2 615 393**
**GB-A- 1 546 283**
**NEFTEKHIMIYA, vol. 19, no.3, 1979 Pergamon Press Ltd, (Poland) G.A. Tolstikov et al.: « Alkylaluminiumdichlorides as catalysts in the addition of hydrogen sulphide to olefins », pages 110-116**

(73) Titulaire : **FIRMENICH SA**
**1, route des Jeunes**
**CH-1211 Genève 8 (CH)**

(72) Inventeur : **Demole, Edouard Paul**
**Chemin des Grands Huttins**
**CH-1296 Coppet/VD (CH)**
Inventeur : **Enggist, Paul**
**39, chemin Moise Duboule**
**CH-1209 Genève (CH)**

(74) Mandataire : **Salvadori, Giuseppe, Dr.**
**c/o Firmenich S.A. Case Postale 239**
**CH-1211 Genève 8 (CH)**

**EP 0 054 847 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a trait au domaine de la parfumerie et à celui des arômes. En particulier, l'invention se rapporte à l'utilisation d'un composé terpénique soufré en tant qu'ingrédient parfumant ou aromatisant.

Il s'agit en l'occurrence du composé de formule

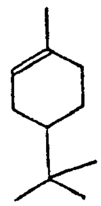

(I)

sous l'une quelconque de ses formes racémique ou énantiomères, soit les composés de formule

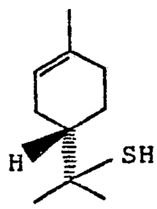 ; 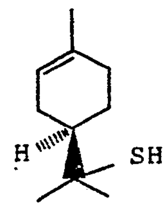 ; 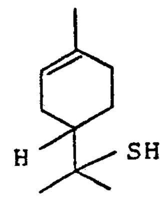

(S)-(-)- (Ia) ; (R)-(+)- (Ib) ; (RS)- (Ic)

Le composé représenté à l'aide de la formule (I) est défini comme le 1-p-menthène-8-thiol, entité chimique qui, sous sa forme racémique, a été décrite une seule fois dans la littérature scientifique [voir : G. A. Tolstikov et al., Neftekhimiya, *19,* 425-9 (1979), tel que résumé dans Chem. Abstr., *91,* 107252 q (1979)], sans toutefois que ses propriétés organoleptiques soient mentionnées ou que son utilisation comme agent parfumant ou aromatisant soit reconnue ou même suggérée.

Nous avons maintenant découvert que non seulement les composés de formule (I) possèdent des qualités olfactives et gustatives fort utiles, mais que leurs propriétés étaient tout à fait exceptionnelles.

Nous avons constaté en effet que ces composés manifestaient une puissance hors du commun, jamais atteinte par des composés parfumants ou aromatisants. Leur seuil de perception dans l'eau est de l'ordre du cent millième de ppb (parties par milliard), plus exactement de $2 \times 10^{-5}$ ppb pour le (R)-(+)-l-p-menthène-8-thiol et $8 \times 10^{-5}$ ppb pour le (S)-(−)-l-p-menthène-8-thiol, ce qui signifie que des effets marqués peuvent se manifester déjà par l'utilisation desdits composés à des concentrations de l'ordre de quelques parties par milliard en poids par rapport au poids des produits dans lesquels ils sont incorporés. Par ailleurs, les caractères positifs propres des composés (I) se déploient à très grande dilution, leur odeur à l'état pur est en effet nettement désagréable, voire nauséabonde.

En termes de puissance, les composés de l'invention se distinguent donc de façon très accentuée de tout composé connu à ce jour.

Quant à la nature de leurs caractères organoleptiques, il est apparu qu'ils apportent une solution originale à un problème qui a occupé depuis fort longtemps l'industrie des arômes, à savoir la reconstitution de l'arôme spécifique du pamplemousse. A cet effet, il faut remarquer que les compositions aromatisantes actuellement sur le marché sont peu satisfaisantes puisqu'aucune n'est en mesure de reproduire, dans les applications envisagées, le caractère gustatif naturel du fruit même, de son jus en particulier.

Ceci est d'autant plus étonnant que nombreuses ont été à ce jour les études consacrées à ce fruit, à l'analyse de ses constituants en particulier.

Par ailleurs, une étude récente a été effectuée par Srivas R. Srinivas [voir le résumé de la communication présentée par cet auteur au Middle Atlantic Regional Meeting de l'American Chemical Society, Mars 1979, Sec. 7].

Cette étude, qui relate l'analyse de l'arôme de pelure de pamplemousse pressée à froid, fait mention de l'influence déterminante sur l'arôme de certains de ses constituants soufrés bicycliques de formule

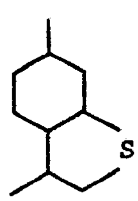 ; 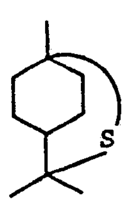 ; 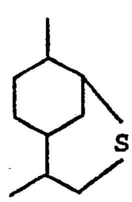 ; 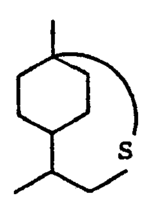 ;

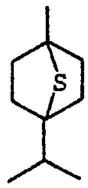

et

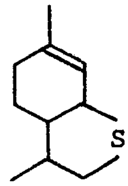

Aucune mention n'a été faite cependant par l'auteur cité des composés particuliers de l'invention.

L'art antérieur fait également état d'un procédé pour la préparation de mercaptans allyliques au moyen de xanthogénate d'allyle [voir DE-OS 26 15393], lequel procédé permet entre autres la préparation d'un mercaptan terpénique de structure analogue à celle des composés (I) de l'invention. Il s'agit en particulier du composé de formule

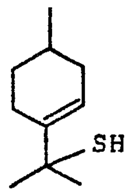

[voir composé (40) à la page 11 du document précité]. Quoique ce document, dans des termes généraux, fasse état du fait que les mercaptans allyliques ainsi préparés possèdent des caractères odorants utiles, de type fleuri et boisé, aucune mention particulière n'a été faite à l'égard du composé ci-dessus indiqué.

En considération de leurs propriétés organoleptiques, les composés (I) trouvent une utilisation fort étendue aussi bien en parfumerie que dans le domaine des arômes, où ils servent à conférer, développer ou améliorer la note odorante ou parfumante typique des agrumes, tout particulièrement du pamplemousse. Ils confèrent de la fraîcheur par exemple aux essences d'orange ou de citron en leur donnant un aspect plaisant de fruit vert.

Des effets surprenants et particulièrement plaisants ont été observés par l'adjonction de l'un des composés (I) à de l'essence de bergamote, à laquelle ils confèrent plus de vie et puissance. Utilisés dans des compositions de type eaux-fraîches, ils jouent un rôle positif, tout aussi bien vis-à-vis des composants de type agrumes, que des composés jasminés ou boisés en leur conférant du montant et de la puissance.

Les proportions dans lesquelles les composés (I) peuvent être utilisés pour la préparation de compositions parfumantes, bases ou concentrés varient dans une gamme de valeurs assez étendue. Cependant, compte tenu de leur puissance, ces proportions sont de l'ordre de quelques parties par million en poids, par rapport au poids de la composition, base ou concentré qui les contient.

Lors d'applications dans le domaine des arômes, ces concentrations peuvent être bien inférieures. C'est ainsi que des effets marqués ont été observés par l'emploi des composés (I) à des concentrations de l'ordre du ppb, comprises par exemple entre 0,001 et 0,010 ppm.

De telles valeurs de concentration ne sauraient être interprétées de façon restrictive. L'homme de l'art sait en effet que des variations de concentration sont nécessaires afin d'accorder au mieux l'effet exercé par les composés de l'invention avec celui des autres ingrédients dans une composition donnée. De telles concentrations dépendent en outre de la nature des articles ou aliments que l'on désire parfumer ou aromatiser.

Les composés de l'invention peuvent être utilisés soit isolément, soit en mélange avec ses dérivés cycliques, l'endo-4,7,7-triméthyl-6-thiabicyclo [3.2.1] octane en particulier, leurs caractères organoleptiques pouvant se compléter harmonieusement dans la plupart des applications pratiques considérées.

Nous avons remarqué que dans certaines conditions de stockage, le 1-p-menthène-8-thiol se transforme en ledit isomère de cyclisation de formule

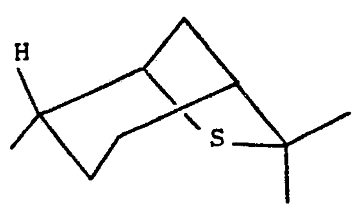

ce qui rend nécessaire l'addition de stabilisants au cas où une longue période de stockage serait à envisager. Comme l'on peut supposer qu'une telle cyclisation est due à un phénomène radicalaire, on peut utiliser comme stabilisants des produits conventionnels tel par exemple le 3-tert-butyl-4-hydroxyanisole (BHA), la tert-butyl-hydroquinone (TBHQ) ou le tocophérol.

Le (RS)-1-p-menthène-8-thiol peut être préparé suivant le procédé décrit par Tolstikov [op. cit.], tandis que les énantiomères (S)-(−) et (R)-(+)- ont été préparés conformément au schéma réactionnel que voici :

Schéma

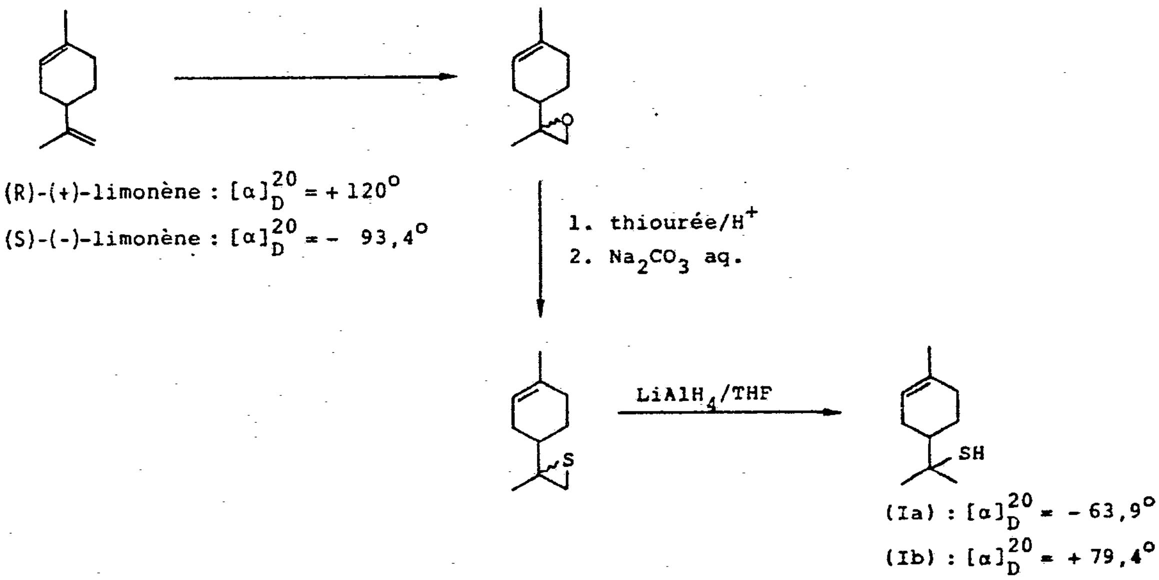


La méthode de préparation particulière suivie est donnée ci-dessous dans le détail.

a. 8,9-Epoxy-1-p-menthène

48,5 g (0,5 M) d'eau oxygénée à 35 % ont été ajoutés goutte à goutte à 40° et en 15 minutes à un mélange constitué par

— 68 g (0,5 M) de limonène [(R)-(+)- ou (S)-(−)-limonène],
— 51,5 g (0,5 M) de benzonitrile,
— 12,5 ml d'une solution aqueuse 0,1 M de $Na_2HPO_4$ et
— 375 ml de méthanol,

puis en 20 minutes, on a ajouté au mélange obtenu, tout en refroidissant extérieurement le réacteur de façon à maintenir la température à environ 40°, 12,5 ml d'une solution aqueuse 0,5 N de NaOH. La réaction reste exothermique durant 25 à 40 minutes après la fin de l'introduction de NaOH. On a ensuite ajouté du NaOH à 5 % afin de maintenir le pH du mélange à environ 9,5-10,0, tout en maintenant la température à 40°. Le progrès de la réaction peut être suivi par analyse gaz-chromatographique. Après dilution à l'eau, le mélange réactionnel a été extrait 3 fois au $CHCl_3$, lavé 3 fois avec du thiosulfate de sodium à 10 %, 1 fois avec KI à 5 %, 1 fois à l'eau, séché sur $MgSO_4$ et concentré à sec sous vide. Le résidu a été repris avec de l'éther de pétrole 30-50, laissé reposer 1 h à 20° et filtré. Par concentration à sec du filtrat, on a obtenu 87,0 g d'un mélange contenant l'époxyde désiré à raison de 27 %.

Un échantillon analytique a été obtenu par distillation fractionnée et chromatographie sur colonne (gel de silice/toluène : EtOAc) :
Eb. 26°/0,133 Pa.
IR : 1 440, 1 175, 910, 840, 790 $cm^{-1}$ ;
SM : $M^+ = 152$ ; m/e : 94, 79, 121 ;
RMN (90 MHz) : 1,28 (3H, s) ; 1,65 (3H, s) ; 1,2-2,3 (7H, m) ; 2,5-2,70 (2H, m) ; 5,35 (1H, s) δ ppm.

b. 8,9-Epithio-1-p-menthène

2,38 g (15,6 mM) de l'époxyde obtenu sous lettre a. ont été introduits goutte à goutte à environ + 5° dans un mélange maintenu sous azote constitué par

— 1,19 g (15,6 mM) de thiourée
— 0,47 ml de $H_2SO_4$ et
— 5,47 ml d'eau.

Le tout a été agité 15 min à environ 0° puis 14 h à 20°.

Après dilution avec 60 ml d'eau, on ajoute goutte à goutte au mélange en 1 h 1,66 g (15,6 mM) de $Na_2CO_3$ dans 8,6 ml d'eau. L'agitation a été poursuivie 20 min. à 25°, puis 25 min. à 50°, après quoi le

mélange a été extrait au pentane (3 x), lavé à l'eau (3 x), séché et évaporé. Par distillation du produit brut, on a obtenu 1,44 g (54 %) du composé désiré.

Eb. 40°/0,133 Pa

IR : 1 430, 1 375, 1 150, 1 055, 915, 785-800 $cm^{-1}$ ;

SM : $M^+$ = 168 ; m/e : 68

RMN (90 MHz) : 1,52 (3H, s) ; 1,66 (3H, s) ; 1,2-2,2 (7H, m) ; 2,30-2,50 (2H, m) ; 5,35 (1H, s) δ ppm.

c. 1-p-Menthène-8-thiol

0,637 g (3,8 mM) du composé obtenu sous lettre b. dissous dans 12 ml de tétrahydrofuranne (THF) anhydre ont été introduits sous azote dans une suspension agitée et portée à reflux de 72 mg (1,9 mM) de $LiA1H_4$ dans 3 ml de THF. Le reflux a été maintenu pendant 1 h, puis après décomposition de l'excès de $LiA1H_4$ avec $H_2O$ à 0°, on a extrait 3 fois à l'éther puis les extraits éthérés ont été lavés à l'eau (3 x), séchés et évaporés à sec. Par distillation fractionnée du résidu, on a obtenu 0,450 g (69 %) du 1-p-menthène-8-thiol désiré.

Eb. 40°/0,133 Pa ;

IR : 1 440, 1 380, 1 360, 1 155, 1 120 et 795 $cm^{-1}$ ;

RMN (90 MHz) : 1,35 (3H, s) ; 1,40 (3H, s) ; 1,56 (1H, s) ; 1,66 (3H, s) ; 1,2-2,2 (7H, m) ; 5,35 (1H, m) δ ppm.

Dans la description du procédé ci-dessus, les températures ont été indiquées en degrés centigrades et les abréviations ont le sens usuel dans l'art.

Comme indiqué plus haut, dans le domaine de l'aromatisation, les composés terpéniques soufrés de l'invention se sont révélés particulièrement adaptés à la reconstitution de l'arôme caractéristique du pamplemousse. Cependant, leurs propriétés gustatives sont telles que l'on peut sans plus envisager la généralisation de leur emploi. Grâce à leur note fruitée, fraîche et montante, lesdits composés peuvent être utilisés pour rehausser la note naturelle de jus de fruit, tel l'abricot, la pêche, le fruit de la passion ou le cassis. Ils servent également à l'aromatisation de boissons de nature variée, les infusions ou les décoctions par exemple, ainsi qu'à l'aromatisation des boissons non nutritives, diététiques et des produits pour l'hygiène buccale. Ils confèrent également des caractères aromatiques fort appréciés aux chewing-gums, aux bonbons ou sucres cuits ou au tabac.

Quoique les propriétés organoleptiques des différents composés de formule (I) soient très analogues, nous avons remarqué que le (S)-(−)-1-p-menthène-8-thiol présentait une note plus fruitée et moins soufrée que le (R)-(+)-1-p-menthène-8-thiol. Leur champ d'application cependant reste tout à fait similaire.

L'invention est illustrée mais non limitée par les exemples suivants.

Exemple 1

3,0 g d'essence de pamplemousse du commerce ont été utilisés afin d'aromatiser 10,0 l de sirop acidulé préparé en dissolvant 6 kg de sucre de canne dans 10 litres d'eau auquel on a ajouté de l'acide citrique à raison de 0,2 %. A deux échantillons de la solution ainsi obtenue, on a ajouté du 1-p-menthène-8-thiol racémique à raison de 0,002 et 0,005 ppm. Les boissons résultantes présentaient un caractère plus montant et plus juteux qui rappelle l'arôme particulier et naturel du jus de pamplemousse.

Le composé racémique peut être remplacé par l'un des énantiomères de formule (Ia) ou (Ib). Des résultats tout à fait similaires ont été observés.

Exemple 2

Certains jus de fruit commerciaux ont été aromatisés à l'aide de 1-p-menthène-8-thiol. Le dosage utilisé ainsi que le résultat de l'évaluation gustative effectuée par un groupe d'experts sont reproduits ci-après.

| | Dosage (ppb) | Commentaires d'évaluation |
|---|---|---|
| - jus d'abricot | 1-2 | plus frais, velouté |
| - jus de pêche | 1-2 | plus montant et plus frais |
| - jus du fruit de la passion | 2-3 | plus frais, plus juteux et typique |
| - jus de cassis | 1-2 | plus juteux, frais et caractéristique |

Exemple 3

Une composition aromatisante de base de type menthe a été préparée en mélangeant les ingrédients suivants :

| Ingrédients | Parties en poids |
|---|---|
| Essence de menthe poivrée | 800 |
| Essence d'orange | 100 |
| Essence de pamplemousse | 50 |
| Essence d'anis | 50 |

On a ensuite préparé un arôme d'essai (A) et un arôme de contrôle (B) en diluant la base « menthe » (10 % dans l'éthanol à 95 %) à raison de 10 g de base pour 100 l de sirop dilué (préparé en dissolvant 1 kg de saccharose dans 600 ml d'eau). La composition (A) a été ensuite aromatisée avec du 1-p-menthène-8-thiol à raison de 2-4 ppb.

Le goût de sirop aromatisé a été trouvé plus montant et plus herbal que celui de l'arôme de contrôle.

Exemple 4

Une composition parfumante a été préparée comme suit (parties en poids) :

| | |
|---|---|
| Essence de bergamote synth. | 200 |
| Essence de mandarine | 50 |
| Aldéhyde mandarine à 10 % * 1) | 5 |
| Essence de lavandin | 50 |
| Myroxyde® 1) | 5 |
| Citronellol synth. | 50 |
| Aldéhyde hexyl cinnamique | 50 |
| Hydroxycitronellal | 100 |
| Mayol® 1) | 10 |
| Mousse d'arbre synth. à 10 % * | 50 |
| Cetyver 1) | 50 |
| Essence de Jonquille à 10 % * | 10 |
| Hédione® 1) | 50 |
| Galaxolide® 2) | 50 |
| Musc DTI 1) | 50 |
| 1-p-Menthène-8-thiol à 1 ‰ | 15 |
| | 795 |

* Dans le citrate d'éthyle
1) Origine : Firmenich SA, Genève — Suisse
2) Origine : Intern. Flavors & Fragrances, USA

Le caractère olfactif de la composition acquiert, grâce à l'adjonction de 1-p-menthène-8-thiol, plus de puissance et de montant.

**Revendications**

1. Composé de formule

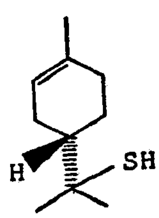

(Ia)

(S)-(-)-

2. Composé de formule

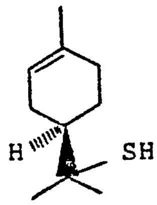

(R)-(+)-

(Ib)

3. Utilisation d'un composé de formule

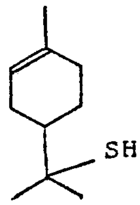

(I)

en tant qu'ingrédient parfumant pour la préparation de parfums, bases ou produits parfumés.

4. Utilisation d'un composé de formule

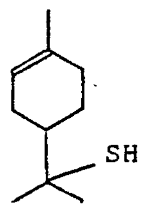

(I)

en tant qu'ingrédient aromatisant pour l'aromatisation d'aliments, de boissons, de chewing-gums, de préparations pharmaceutiques ou de tabac.

5. Composition parfumante contenant en tant qu'ingrédient actif un composé de formule (I) suivant la revendication 3.

6. Composition aromatisante contenant en tant qu'ingrédient actif un composé de formule (I), suivant la revendication 4.

7. Procédé pour conférer, améliorer ou modifier le caractère gustatif propre du pamplemousse dans les aliments ou les boissons, caractérisé en ce qu'on ajoute auxdits aliments ou boissons un composé de formule (I), suivant la revendication 4.

**Claims**

1. Compound of formula

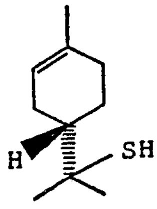

(S)-(-)-

(Ia)

2. Compound of formula

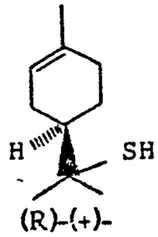

(R)-(+)-

(Ib)

3. Use of a compound of formula

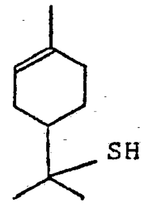

(I)

as perfuming ingredient for the preparation of perfumes, bases or perfumed products.

4. Use of a compound of formula

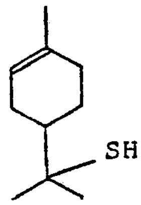

(I)

as flavouring ingredient for the aromatization of foodstuffs, beverages, chewing-gums, pharmaceutical preparations or tabacco.

5. Perfuming composition containing as active ingredient a compound of formula (I) according to claim 3.

6. Flavouring composition containing as active ingredient a compound of formula (I) according to claim 4.

7. Method to confer, improve or modify the characteristic gustative feature of grapefruit in foodstuffs or beverages, characterized in that there is added to said foodstuffs or beverages a compound of formula (I), according to claim 4.

**Patentansprüche**

1. Verbindung der Formel

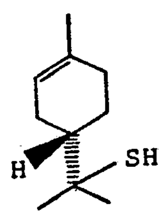

(Ia)

(S)-(-)-

2. Verbindung der Formel

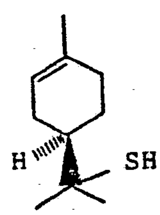

(Ib)

(R)-(+)-

3. Verwendung einer Verbindung der Formel

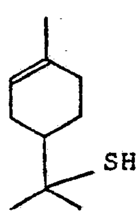

(I)

als Parfümbestandteil zur Herstellung von Parfümen, Parfümbasen oder parfümierte Produkten.

4. Verwendung einer Verbindung der Formel

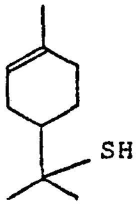

(I)

als Aromabestandteil für die Aromatisierung von Nahrungsmitteln, Getränken, Kaugummi, pharmaceutischen Präparaten oder Tabak.

5. Parfümkomposition, welche, als aktiven Bestandteil, eine Verbindung der Formel (I) gemäss Anspruch 3, enthält.

6. Aromakomposition, welche, als aktiven Bestandteil, eine Verbindung der Formel (I) gemäss Anspruch 4, enthält.

7. Verfahren um den der Grapefrucht eigenen Geschmackscharakter in Nahrungsmitteln oder Getränken zu erhalten, zu verbessern oder zu modifizieren, dadurch gekennzeichnet, dass man den genannten Nahrungsmitteln oder Getränken eine Verbindung der Formel (I) gemäss Anspruch 4, zufügt.